# EUROPEAN PATENT SPECIFICATION

(11) **EP 1 747 002 B1**
(45) Date of publication and mention of the grant of the patent: **14.10.2009**
(21) Application number: 05745024.9
(22) Date of filing: 13.05.2005
(51) Int. Cl.: A61K 31/616, A61P 33/04

(54) **NEW APIARY VETERINARY COMPOSITION**
VETERINÄRE ZUSAMMENSETZUNG FÜR BIENEN
NOUVELLE PREPARATION VETERINAIRE APICOLE

(30) Priority: 17.05.2004 HU 0400982
(43) Date of publication of application: 31.01.2007
(73) Proprietor: Chemor Kutato, Fejleszto es Kereskedo Kft., 7100 Szekszard (HU)
(72) Inventor: ORBÁN, Gyula, H-7100 Szekszárd (HU)
(74) Representative: Szentpéteri, Adam
(86) International application number: PCT/HU2005/000053
(87) International publication number: WO 2005/110384

(56) References cited:
- US-A- 4 859 658
- DATABASE WPI Section Ch, Week 200116 Derwent Publications Ltd., London, GB; Class B05, AN 2001-157202 XP002351533 & RU 2 153 327 C2 (UNIV FAR E AGRIC) 27 July 2000 (2000-07-27)
- SAMSINAKOVA A ET AL: "EFFECTS OF SOME ANTI MYCOTICS AND DISINFECTANTS ON THE ASCOSPHAERA-APIS FUNGUS IN-VITRO" ZEITSCHRIFT FUER ANGEWANDTE ENTOMOLOGIE, vol. 84, no. 3, 1977, pages 225-232, XP008054809 ISSN: 0044-2240
- DATABASE CA [Online] CHEMICAL ABSTRACTS SERVICE, COLUMBUS, OHIO, US; GONTARSKI, H. ET AL: "Quantitative studies on the chemotherapeutic treatment of the honey bee against Nosema apis" XP002351526 retrieved from STN Database accession no. 1954:50494 & ARZNEIMITTEL-FORSCHUNG , 4, 161-8 CODEN: ARZNAD; ISSN: 0004-4172, 1954,
- DATABASE BIOSIS [Online] BIOSCIENCES INFORMATION SERVICE, PHILADELPHIA, PA, US; 2000, MLADJAN V ET AL: "Preventive action of fumagillin on the degree of infection with Nosema apis in the digestive tract of bees" XP002351527 Database accession no. PREV200100017927 & ACTA VETERINARIA (BELGRADE), vol. 50, no. 4, 2000, pages 241-252, ISSN: 0567-8315

## Description

The subject of the invention is a new apiary veterinary composition, for use in the treatment and/or prevention of the nosema disease (*Noseniosis apium*) at honey-bees (Apis mellifera).

It is known that this extremely dangerous disease of the honey-bees (Apis mellifera) is caused by a spore amoeba called *Nosema apis.* The main period of the disease is in spring, although the disease itself can occur at other times, too. The content of the rectum of the healthy winter bees increases only slowly in the first half of the winter. During the last third of the winter a sudden increase occurs, because the commenced brood raising forces the otherwise healthy bees to increase their metabolism. The content of the bees infected with nosema increases gradually, because the water content of the intestine increases, this adds to the otherwise tense content of the rectum and consequently the ill individuals start defecating within the hive. The excrements get on the bees, on the walls of the cells, on the cell-caps, pollen, and on the honey. The healthy members of the colony get infected due to their cleaning instinct. The cleaning bees suck the thin excrement and they lick the dried excrement, too, after previously moistening it. During this activity the bees get infected with huge amounts of *Nosema apis* spores.

These spores keep their life-functions within the excrements defecated by the bees for at least one year. The excrement residues dried on the comb can consequently infect the bees one year later. The deadly effect of the amoeba shows itself mainly in the destruction of the epithelium of the midgut of the bees, but it causes other abnormalities as well. For instance the pharyngeal gland of the bees develops vestigially, and whereas in case of healthy colonies only 6% of the bees have vestigial pharyngeal glands, in case of bees with nosema this proportion can reach 41%. The vestigial pharyngeal gland is not able to produce enough royal jelly and consequently the malnutrition of the brood occurs, or the brood can even starve to death , underdeveloped bees hatch with incomplete wings. As the proteins for the brood comes from the pharyngeal gland, it can be understood that the *Nosema apis* infection has its effect upon the brood raising. The untreated colonies are completely destroyed in most cases, thus the prevention or the treatment of the infected colonies is indispensable.

The disease is not easy to be diagnosed based on the symptoms, but the experienced specialist is able to observe the typical symptoms. (For example in spring one can see walking bees, which are unable to fly and vibrate their outstretched wings. The ill worker walks weakly on the flight board and it falls easily on the ground in front of the hive. It climbs on the grass with vibrating wings and it tries to fly, but it falls back weakly. In the hive, on the comb and on the wood of the frames several excrement marks can be seen. The deserted hive is left by the queen, too, and in these severe cases the queen herself can be seen walking on the ground. If the queen is infected with nosema, it ceases egg laying and it dies within one or two weeks). The secure diagnosis of the disease involves laboratory examination, which is based on the determination of the spores. The successful determination of the pathogen in hugely influenced by the method of taking of the samples.

Due to the severity of the nosema disease more protective methods are known. A well-probed apiary complementary method is the disinfection of the hive and the comb with acetic acid. For the disinfection technical acetic acid is to be used. This has a concentration of 80%, so it is fluid under the temperature of 15 °C . In comparison the 100 % concentrated ice-acid gets solid under 15 °C. During the disinfection the calculated amount of technical acetic acid is poured into a porcelain bowl and put into the hive to be disinfected. The main disadvantage of the method is that the disinfected combs have to be aired for several days after usage, otherwise acid steams will lead to brood and bee destruction. A further disadvantage is that the vapour of the acetic acid have a negative effect on the metal objects. Thus it significantly diminishes the life-span of these of the hives and accessories.

In the past the compositions with mercury content were extensively used in the treatment of the *Nosemosis apis*. The mercury content of these compositions endangered the honey, as a food-composition, and consequently these substances with mercury content were completely withdrawn from usage.

To the withdrawal of the mercury-containing compositions the introduction of a much more effective composition had its role, namely the antibiotic which in Hungary was introduced with the name Fumagillin DCH. This antibiotic was first used by Canadian researchers for the treatment of the nosema disease. The active ingredient of the medicine is the antibiotic got from the *Aspergillus fumigatus* mould, which was basically used for the treatment of the human dysentery caused by amoebae in tropical countries. After its efficacy in the treatment of the *Nosema apis* was discovered, its usage has spread quickly all over the world.

The Fumagillin DCH pulvis produced by Sanofi-Chinoin Drug Manufacturing Company Budapest, Hungary is marketed in bottles containing 20 g. One bottle contains 0.5 g of pure active substance. According to the instructions of usage the content of one bottle is sufficient for the treatment of 10 mildly or 5 severely infected colonies. Before the usage the content of the bottle has to be dissolved in 0.5 litres of tepid water, then is evenly mixed in 5 litres of sugar syrup in proportion of 1:1 cooled to 40 °C, and then the mixture is diluted to 25 litres with sugar syrup. The ill colonies are fed with 0.5 litres of the mixture daily. The usage of the compositions containing antibiotics is more and more restricted and even forbidden by the authorities of certain countries.

The aim of the invention producing of such an effective -and if possible containing natural ingredients- medicine composition, with which the above mentioned disadvantages can be avoided and the nosema disease can be treated with good effectiveness or it can be prevented.

The invention is based on the observation that according to the apiary experiences the nosema disease diminishes and even ceases in case of the visiting of bee pastures with high occurrence of willow. After the examination of several other kinds of plants and flowers my attention was attracted by these ones. I determined that according to the literature in the pussy willow of the white willow (Salix alba), but especially in the buds of the black poplar (Populus nigra) an important amount of 2-hidroxibenzil-β-D-glucopiranozid (salicin) can be found. This compound can be considered a precursor of the salicylic acid. This is because I presume that the salicin transforms into salicylic acid in the midgut of the bee (at the effect of acid, enzyme and given temperature), and this is how it can have its anti-nosema effect.

Surprisingly enough the supposition was completely proven by several in vivo experiment series. The effective amount of salicylic acid, as the effective active substance concentration was determined again based on more experiment series. The deduction drawn was thus that a composition containing a certain salicylic acid or salicylic acid derivative concentration, completed with adequate carrying substance was effective even in case of extreme infections with nosema disease at bee colonies. This recognition could not have been foreseen by any means, because the among the multilateral forms of usage of the salicylic acid and acetyl salicylic acid (for example anti-bacterial, antiseptic, and even anti-rheumatic effect) the apiary usage was not mentioned anywhere

Based on the above the invention is such a veterinary composition, which is for use for the treatment and/or prevention of the nosema disease (Nosemosis apium) at honeybees and which contains as active substance 2-hydroxy benzoic acid (salicylic acid) or 2-acetoxy benzoic acid (acetyl- salicylic acid) or the salt of these.

The composition according to the invention contains in an advantageous form sugar-industry molasses as carrier matter (concentrated watery extract of Beta vulgaris cv altissima), and if necessary, other known auxiliary and carrier substance.

The composition in its advantageous form contains basically two main components. The main active substance is the 2-hydroxy benzoic acid (salicylic acid), which kills the parasite spore amoebae. The other component is the carrier substance Beta vulgaris cv altissima in concentrated fractionated watery extract (sugar-industry molasses), which contains such natural components (macro- and micro-elements, vitamins), which partly diminish the shortages caused by sugar-feeding (alimentary saccharine), so the strengthened colonies can defend themselves more effectively against diseases, thus against the nosema disease as well.

My experiences show that the effect of the composition based on the invention is present 4 weeks after the treatment too, and furthermore according to the laboratory tests done during the following spring the colonies treated the previous autumn were free of the nosema spores which cause the infection.

The tests done with the composition based on the invention produced excellent results and the composition proved to be effective in all the phases of the disease (in the low, medium, high, and very high infected categories). The comparing experimental results are shown among the examples. It is important to note that due to its components, the advantageous carrier substance (concentrated fractionated watery extract of Beta vulgaris cv altissima, sugar-industry molasses) supplements shortages induced by the usual sugar feeding (macro-, and micro-elements, vitamins).

The composition based on the invention, beyond its main effect, enhances certain life activities of the colonies, for example the honey flow and the cleaning willingness, together with the brood willingness, thus indirectly helps the prevention and fight against the diseases. This positive side-effect was noticed by the bee-farmers who used the composition based on the invention.

The composition based on the invention, its usage and advantageous effects are shown as follows:

### Example 1.

50,0 ml concentrated fractionated watery extract of Beta vulgaris cv altissima (sugar-industry molasses); carrier substance
10,0 g sodium salt of 2-hydroxy benzoic (sodium salicylic acid); active substance,
ad 100,0 ml distilled water, post-sterilised with ultra-violet rays The drug industry form, that is watery solution.
The manufacturer possesses quality control system and its laboratory checks the quality of the composition regularly.

### Short description of the packing:

Plastic bottle with safety cap (the bottle is white, the cap is green, the material used is high-pressure polyethylene).
Quantity: minimum: 900 ml
   maximum: 1010 ml
On the side of the bottle there is a printed label .
The marketing name of the composition: NONOS

### Example 2.

**Table 1. Cumulative table**

| Of the in vivo experiments related to the experimental composition suitable for the prevention and treatment of the installed nosema (Nosemosis apis) disease at honey-bees (Apis mellifera) | | | | | | | | | | |
|---|---|---|---|---|---|---|---|---|---|---|
| Date 16^{th} March, 2003 - 3^{rd} April, 2003 | | | | | | | | | | |
| Colonies involved in the experiment: | | | | | | | | | | |
| Marking and the sign and quantity of the substance used in the experiment | K₁ NONOS 50 ml | K₆ NONOS 50 ml | K₇ NONOS 50 ml | K₈ NONOS 50 ml | K₁₃ NONOS 50 ml | K₁₄ NONOS 50 ml | K₁₆ NONOS 50 ml | K₁₇ NONOS 50 ml | K₁₉ NONOS 50 ml | K₂₀ NONOS 50 ml |
| Status before the experiment | Very high number of Nosema apis spores | Medium number of Nosema apis spores | Medium number of Nosema apis spores | Medium number of Nosema apis spores | High number of Nosema apis spores | High number of Nosema apis spores | High number of Nosema apis spores | High number of Nosema apis spores | High number of Nosema apis spores | Medium number of Nosema apis spores |
| Status after the experiment | negative | negative | negative | negative | negative | negative | negative | negative | negative | negative |
| Status 4 weeks after the experiment | negative | negative | negative | negative | negative | negative | negative | negative | negative | negative |

### Example 2.

**Table 2. Cumulative table**

| Of the in vivo experiments related to the experimental composition suitable for the prevention and treatment of the installed nosema (Nosemosis apis) disease at honey-bees (Apis mellifera) | | | | | | | | | | |
|---|---|---|---|---|---|---|---|---|---|---|
| Date: 16^{th} March 2003 - 3^{rd} April 2003 | | | | | | | | | | |
| Colonies involved in the experiment: | | | | | | | | | | |
| Marking and the | S₂₂ | S₂₃ | S₂₄ | S₂₅ | S₂₈ | S₂₉ | S₃₁ | S₃₂ | S₃₄ | S₃₅ |
| sign and quantity of the | Fumagillin DCH | Fumagillin DCH | Fumagillin DCH | Fumagillin DCH | Fumagillin DCH | Fumagillin DCH | Fumagillin DCH | Fumagillin DCH | Fumagillin DCH | Fumagillin DCH |
| substance used in the experiment | 2 g | 4 g | 4 g | 4 g | 4 g | 2g | 4 g | 2 g | 4 g | 4g |
| Status before the experiment | Low number of Nosema apis spores | Medium number of Nosema apis spores | Low number of Nosema apis spores | High number of Nosema apis spores | Very high number of Nosema apis spores | Low number of Nosema apis spores | High number of Nosema apis spores | Low number of Nosema apis spores | Medium number of Nosema apis spores | Medium number of Nosema apis spores |
| Status after the experiment | negative | negative | negative | negative | negative | negative | negative | negative | negative | negative |
| Status 4 weeks after the experiment | negative | negative | negative | negative | negative | negative | negative | negative | negative | negative |

### Example 2.

**Table 3. Cumulative table**

| Of the in vivo experiments related to the experimental composition suitable for the prevention and treatment of the installed nosema (Nosemosis apis) disease at honey-bees (Apis mellifera) | | | | | | | | | | |
|---|---|---|---|---|---|---|---|---|---|---|
| Date: 16^{th} March 2003 - 3^{rd} April 2003 | | | | | | | | | | |
| Colonies involved in the experiment: | | | | | | | | | | |
| Marking and the sign and quantity of the substance used in the experiment | K₂ | K₃ | K₄ | K₅ | K₉ | K₁₀ | K₁₁ | K₁₂ | K₁₅ | K₁₈ |
| | Untreated colony | Untreated colony | Untreated colony | Untreated colony | Untreated colony | Untreated colony | Untreated colony | Untreated colony | Untreated colony | Untreated colony |
| | Low number of Nosema apis spores | Medium number of Nosema apis spores | Medium number of Nosema apis spores | High number of Nosema apis spores | Medium number of Nosema apis spores | Low number of Nosema apis spores | Low number of Nosema apis spores | Medium number of Nosema apis spores | Medium number of Nosema apis spores | Medium number of Nosema apis spores |
| Status after the experiment | High number of Nosema apis spores | High number of Nosema apis spores | High number of Nosema apis spores | Very high number of Nosema apis spores | Very high number of Nosema apis spores | Medium number of Nosema apis spores | Medium number of Nosema apis spores | High number of Nosema apis spores | Very high number of Nosema apis spores | Very high number of Nosema apis spores |
| Status 4 weeks after the experiment | The colony died | The colony died | The colony died | The colony died | The colony died | The colony died | The colony died | The colony died | The colony died | The colony died |

### Example 3.

The colonies access to macro- and micro-elements Treatment according to Example 1. for 10 days, and during 10 days of feeding with honey

| | Treatment according to Example 1. (50 ml) | Honey-feeding for 10 (5 kg honey) 100 % |
|---|---|---|
| | | |
| K | 1 g (100 %) | 1 g |
| Ca | 0,1 g (10 %) | 0,25 g |
| Na | 0,2 g (200 %) | 0,1 g |
| Mg | 0,01 g (10 %) | 0,1 g |
| P | 0,01 g (5,6 %) | 0,18 g |
| Fe | 0,002 g (20 %) | 0,01 g |
| Mn | 0,001 g (50 %) | 0,002 g |
| Cu | 0,001 g (50 %) | 0,002 g |

### Example 4.

The colonies access to vitamins with the treatment according to Example 1 for 10 days, and according to the quantities found as proposed in the literature.

| | Treatment according to Example 1. (50 ml) | Necessity (according to the literature) 100% |
|---|---|---|
| | | |
| B₁ vitamin | 0,1 mg (5,6 %) | 1,8 mg |
| B₆ vitamin | 0,2 mg (20 %) | 1,0 mg |
| Nicotine acid | 1,2 mg (3,3 %) | 36 mg |
| Folic acid | 0,5 mg (100 %) | 0,5 mg |
| Biotin | 0,01 mg (20 %) | 0,05 mg |

According to the examples above (see Example 3. and 4.) it can be seen that following the treatment with NONOS, the bee-colonies get an important quantity of macro- and micro-elements, together with vitamins, which increases their capacity of defending themselves against diseases, thus the effectiveness of the product is improved.

### Example 5.

We did the same as in Example 1, with the only difference that instead of the 10.0 g of sodium salt of 2-hydroxy benzoic acid we weighed 12.6 g of acetylsalicylic acid sodium.

## Claims

1. Use of 2-hydroxy-benzoic acid (salicylic acid), 2-acetoxy-benzoic acid (acetylsalicylic acid) or the salts thereof in preparing compositions for the treatment and/or prevention of the nosema disease (Nosemosis apium) of honey bees.

2. The use according to claim 1 wherein the composition comprises sugar-industry molasses, (a concentrated aqueous extract of Beta vulgaris cv altissima) and optionally additional auxiliaries and carriers.

## Patentansprüche

1. Verwendung von 2-Hydroxybenzoesäure (Salizylsäure), 2-Acetoxybenzoesäure (Acetylsalizylsäure) oder deren Salze zur Herstellung von Kompositionen zur Behandlung und/oder Prävention von Nosema Krankheit (Nosemosis apium) von Honig-Bienen.

2. Verwendung nach Anspruch 1 **dadurch gekennzeichnet, dass** die Kompositionen Melassen aus der Zuckerindustrie (eine konzentrierter wässriger Extrakt von Beta vulgaris cv altissima) und gewünschtenfalls weitere Hilfstoffe und Trägerstoffe enthalten.

## Revendications

1. Utilisation de l'acide 2-hydroxybenzoïque (l'acide salicylique), de l'acide 2-acétoxybenzoïque (l'acide acétylsalicylique) ou de sels des ceux-ci pour la fabrication d'un médicament utile dans le traitement thérapeutique et/ou prophylactique de la nosémose (Nosemosis apium) des abeilles.

2. Utilisation selon révendication 1 dans laquelle la composition contient de la mélasse de l'industrie sucrière (l'éxtrait concentré aqueux de la Beta vulgaris cv altissima) et éventuellement des éxcipients and des véhicules supplémentaires.
